# EUROPEAN PATENT SPECIFICATION

(11) **EP 1 232 136 B1**
(45) Date of publication and mention of the grant of the patent: **19.10.2005**
(21) Application number: 00955404.9
(22) Date of filing: 08.08.2000
(51) Int. Cl.: C07C 51/41, C07C 63/26, C07C 63/28, C07C 63/15, C07C 63/14

(54) **ALKYL CARBOXYLATE SALTS AS SOLVENTS FOR HENKEL-RELATED PROCESSES**
ALKYL-CARBOXYLAT-SALZE ALS LÖSUNGSMITTEL FÜR HENKEL-VERWANDTE PROZESSE
SELS ALKYL CARBOXYLATE COMME SOLVANTS UTILISES DANS DES PROCEDES DE TYPE HENKEL

(30) Priority: 30.08.1999 US 151605 P
(43) Date of publication of application: 21.08.2002
(73) Proprietor: Mossi & Ghisolfi International S.A., 1528 Luxembourg (LU)
(72) Inventor: BROWNSCOMBE, Thomas, F., Houston, TX 77005 (US); PFREHM, Susan, Houston, TX 77077 (US)
(74) Representative: Gerbino, Angelo
(86) International application number: PCT/US2000/021648
(87) International publication number: WO 2001/016077

(56) References cited:
- US-A- 2 823 231
- US-A- 3 043 846
- US-A- 3 337 498
- US-A- 4 271 316
- US-A- 4 820 868

## Description

This invention relates to solvents for use in the Henkel disproportionation/isomerization reaction. More particularly this invention relates to unexpected advantages provided by the use of a certain class of compounds as solvents for the Henkel reaction. In another aspect this invention relates to the unexpected production of new methylated diacids and to the concept of manipulating the conditions of the reaction to optimize products with different numbers of methyl groups and different isomers.

### Background Art

Naphthalene monocarboxylic acid and naphthalene dicarboxylic acids other than 2,6-naphthalene dicarboxylic acid can be converted to 2, 6-NDA using a disproportionation reaction in the case of the monocarboxylic acids or a rearrangement reaction in the case of other naphthalene dicarboxylic acids. Henkel and Cie first patented a reaction of naphthoic acid salts to 2,6-NDA in the late 1950s. (See, for example, U.S. Pat. Nos. 2,905,709, 2,863,913, 2,846,468, 2.841,615, 2,823,230, 2,823,229, and 2,794,830.)

The Henkel process, for the production of terephthalic acid (TPA) and purification to purified terephthalic acid (PTA), by reaction of benzoic acid with potassium carbonate at 400-500°C in the presence of CO₂, suffers from the fact that all the materials present, including the starting materials, products, and reactants, are essentially infusible solids. Therefore, mixing and handling are a problem. This problem can be expected to limit conversions and decrease the rate of reaction.

Patents in the art have claimed improvements in particular aspects of the rearrangement type of reaction, however there has been minimal research found in the art relating to the solvent or reaction medium used in the Henkel reaction. U.S. Pat. No. 4,820,868 claims improvements in yield of 2,6-naphthalene dicarboxylic acid for specific isomers subjected to a rearrangement reaction using an aromatic reaction medium.

In U.S. Pat. No. 4,208,536, to Rhone Poulenc, there is disclosed the use of reaction mediums which are stable under reaction conditions, however no "true solvents" for a disproportionation type reaction have been found in the art.

It would be a distinct advance in the art if there were identified an improved solvent for a Henkel type reaction and related processes for purified terephthalic acid that would give optimum partitioning of reactants and products.

It would be an additional unexpected advance in the art if a new aromatic compound were discovered that would be useful in making polyesters.

### Disclosure of the Invention

In accordance with the foregoing the present invention relates to a process having the features indicated in claim 1 which follows. Preferred features of the invention are indicated in the dependent claims.

### Detailed Description of the Invention

In the present invention molten salts are used as solvents for the Henkel reaction. These solvents have several advantages, including being comparatively non-corrosive, stable at high temperatures, and, indeed, promoting the reaction to the extent they do decompose. In addition, the salts have a high dielectric constant, stabilizing the reactive anions and allowing them to more readily form, increasing the rate of the reaction, especially if the acid salt has for its cation an active catalytic Group IIB, Group IIIB, or VIIIA metal.

By the use of such solvents, the contact between the reagents of any of the Henkel I, Henkel II, or Henkel-related processes may be improved, with improved rates and yields. By choosing the acid salt carefully, the right amount of catalytic metal may be incorporated, and the desired partitioning of the salt in the final purification step (e.g. KOAc will be soluble in water; K undecanoate will not be) as well as the correct solvent properties during the reaction (e.g. acetate, propionate, succinate will be more polar, higher melting, and more solvating while higher carbon number salts will be less so) may be obtained to optimize the particular process.

Starting materials for a disproportionation in which the catalyst of the invention is useful include salts of aromatic mono-, di-, or polycarboxylic acids. Such acids include, for example, benzoic acid, α- and β-naphthoic acid, diphenyl monocarboxylic acids, as well as phthalic acid, isophthalic acid, terephthalic acid, naphthalic acid and other naphthalene dicarboxylic acids or diphenic acid and other diphenyl dicarboxylic acids. In addition, mono- or dicarboxylic acids in which the carboxylic groups are attached to another aromatic ring system, for example to anthracene, terphenyl, diphenyl methane or benzophenone radicals, are suitable for use as starting materials for the process of the invention, as well as tri- and polycarboxylic acids which are derived from aromatic ring systems. Also, mixtures of such acids which are formed, for example, by oxidation, or mixtures of alkyl aromatic compounds may be used.

The starting materials may also be salts of monobasic heterocyclic carboxylic acids, the carboxyl groups of which are attached to heterocyclic rings having an aromatic structure. Such acids are derived, for example, from pyridine, pyrazine, pyrimidine, pyridazine, α-pyran, furan, thiophene, thiazole, quinoline, isoquinoline, indole, benzotriazole and benzimidazole.

In all of these carboxylic acids the aromatic ring or the heterocyclic ring having an aromatic structure can, in addition to the carboxyl group, also carry other substituents such as halogen atoms or alkyl radicals, provided that they do not decompose at temperatures below the reaction temperature. The term aromatic carboxylic acid is intended to include both compounds having a homocyclic aromatic ring and compounds having a heterocyclic ring.

The preferred starting materials are aromatic monocarboxylic acids or asymmetrical aromatic dicarboxylic acids.

When aromatic monocarboxylic acids are used as starting materials for a disproportionation reaction, the reaction products obtained are industrially valuable dicarboxylic acids or the salts thereof, such as, for example, terephthalic acid and 2,6-naphthalene dicarboxylic acid.

Aromatic monocarboxylate includes benzoate, methyl benzoate, naphthoate, and similar compounds.

It is advantageous to use the above-mentioned carboxylic acids in the form of an alkali metal salt. Preferably the potassium salts or the sodium salts are used. The lithium, rubidium and cesium salts, may be used, but generally are not for reasons of economy. It is also possible to use mixtures of salts of two different metals. Reaction materials that form the above-mentioned salts may also be used.

Any catalyst that can be used for disproportionation, such as, for example, in the Henkel process could be used in the process of the present invention. Suitable catalysts can be selected from compounds of Group IB, IIB, VB, or VIIB of the Periodic Table. Generally, a suitable catalyst would be selected from zinc compounds, cadmium compounds, and mercury compounds in the form of, for example, oxides, halides, sulfates, carbonates, and carboxylates of these metals.

In the present integrated process a zinc compound is preferred. Suitable zinc compounds include zinc halides such as zinc fluoride, zinc chloride, zinc bromide, and zinc iodide; zinc carboxylates such as zinc naphthoate and zinc naphthalene-dicarboxylate; zinc oxide, zinc carbonate; zinc sulfate and mixtures thereof. Note zinc naphthoate includes a 1-isomer, a 2-isomer, and mixtures thereof, and zinc naphthalenedicarboxylate includes a 1,2-isomer, a 1,3-isomer, a 1,4-isomer, a 1,5-isomer, a 1,6-isomer, a 1,7-isomer, a 1,8-isomer, a 2,3-isomer, a 2,6-isomer, a 2,7-isomer, and mixtures thereof. In the preferred embodiment the catalyst employed was ZnO.

The molten salt solvent according to the present invention may be any molten salt known in the art, but particularly an alkali or alkaline earth metal, or a mixture of two or more of such salts, that has a sufficiently low melting point to dissolve the other components of the reaction mixture without thermal decomposition, and which is sufficiently inert under the reaction conditions not to react with the other components of the reaction mixture. As used in the present application, the alkali metals include the elements of Group IA of the Periodic Table and the alkaline earth metals are the elements of Group IIA of the Periodic Table. The salts have a melting point of about 500°C or less.

The molten salt solvent may also have a minor amount of another salt, i.e., not an alkali or alkaline earth metal salt, preferably derived from the same acid anion. In accordance with the present invention, this "minor amount" of another salt is at most 50% by weight of another salt.

Salts of sodium and potassium are particularly suitable alkali metal salts for use in the present invention, although other alkali metal salts, such as salts of lithium, may also be used. Salts of magnesium and calcium are particularly suitable alkaline earth metal salts for use in the present invention, although salts of other alkaline earth metals such as strontium and barium may also be used in the practice of the present invention. However, the use of alkaline earth salts will favor 2,3-NDAs over 2,6-NDAs and 1,2- over 1,4-benzene diacids.

According to the present invention, the alkali or alkaline earth metal salts used are derived from an inorganic or an organic acid. Inorganic acids from which the salt may be derived include phosphoric acid, and nitric acid. Suitable organic acids include aliphatic carboxylic acids, such as, for example acetic, and fatty acids with up to 18 carbon atoms per molecule.

Preferred are salts that are basic or at least have basic elements, such as, but not limited to the inorganic salts of carbonic acid, alkoxides, and carboxylates. Phosphates work, but are less preferred. Halides would work in the reaction, but lead to metallurgical problems. Sulfates lead to decarboxylation.

The most preferred salts mixtures useful in the molten salt solvent of the present invention can be derived from carbonic acid. Suitable examples include alkali metal carbonates. The alkali metal can be selected from the group consisting of lithium, sodium, potassium, rubidium, and cesium, but is preferably potassium. Carbonates which can be used include, but are not limited to, K₂CO₃, KHCO₃, Rb₂CO₃, RbHCO₃, Cs₂CO₃, CsHCO₃, and other strongly basic carbonates or bicarbonates. We have found it advantageous to use potassium carbonate or potassium bicarbonate.

It is preferred that the salt mixtures have, at least approximately, a eutectic composition. A eutectic mixture provides the lowest melting point of a mixture of two or more alkali metals that is obtainable by varying the percentage of the components. Eutectic mixtures have a definite minimum melting point compared with other combinations of the same metals. For example, though the melting point of Li₂CO₃ is 622°C, in a eutectic mixture of alkali carbonates the melting point can be 400°C. What is required, where a eutectic mixture is employed, is the right mixture of alkali metal carbonates where the melting point is less than about 400°C. Generally the ratio of alkali metal carbonates in the eutectic mixture is about 1:1:1, but it can vary.

In the practice of the present invention a particularly preferred salt mixture is selected from K₂CO₃, Rb₂CO₃, Cs₂CO₃, and Na₂CO₃ and mixtures thereof.

The salts used to form the molten salt solvent in the practice of the present invention should be substantially free of water. The amount of water present should be less than or about 1000 ppm. Although too much water can present problems, it has been observed that a small amount of water seems to actually be beneficial. It is speculated the beneficial effects are due to the effect of the water of introducing increased mobility into the salts, specifically allowing the salt complexes more rotational freedom and also by stabilizing the charged species formed as intermediates. However, a significant amount of water, say, for example in excess of ca. 700-1000 ppm, interferes with the reaction by beginning to favor the decomposition of the salts (decarboxylation). An amount of water of 0.2% or more becomes damaging by promoting yield loss.

Suitable temperatures for the disproportionation reaction are in the range of from about 340°C to 500°C. Better results are observed where the temperature is from about 360°C to 480°C. The preferred temperature is from about 400°C to 460°C. At 450°C a significant yield of TPA was observed.

Another aspect of the present invention comprises new monomer diacids prepared by a new reaction, the transfer of methyl groups from potassium acetate to diacid salts in the acid state. By this means, methylated diacids, especially symmetrically methylated diacids, may be produced. Thus potassium acetate, which melts (Condensed Chemical Dictionary, 6th edition) at 292°C, was tried as a molten reaction medium for the Henkel reaction of potassium 2-naphthoate or potassium benzoates with and without ZnO as catalyst. It was thought that since the salt melts at a lower temperature than the carbonate eutectics, it might be possible to perform the reaction at a lower temperature, however, little Henkel reaction was observed at temperatures significantly below 400°C. Unexpectedly, however, on examining the reaction products of the black fused mass formed from the potassium acetate salt and the naphthoic or benzoate potassium salts at ca. 400°C and above, a significant portion of methylated diacids was observed. The reaction favored the production of the diacids in a temperature range of about 400-500°C, preferably 450°C.

Additionally, it was found that increasing the time of reaction resulted in increasingly higher yields of the methylated compounds, and an increasingly higher proportion of tetramethylated TPA. It was possible to selectively form the dimethyl TPA by shorter reaction time and milder temperatures, and the tetramethyl by longer reaction time and higher temperatures. To produce tetramethyl (permethyl) a temperature of about 450°C or more, and times of at least 2 hours are required. The monomethyl was not the major product at either time. This result implies an added stability, by phase separation or for other reasons, to the dimethyl material, which, when driven, produces the tetramethyl material as the final product. It will be appreciated by those skilled in the art that a series of contacts and process steps can be designed to result in highly selective production of the dimethyl or tetramethyl TPA, since the lesser methylated materials can, after separation and, if necessary, purification, be recycled for further reaction ultimately forming the desired dimethyl or especially tetramethyl species.

Those skilled in the art will appreciate that for each salt mixture, base amount, etc. a given reactivity pattern will appear, so the time and temperature will be different, but in all systems, high severity for that system will result in the complete methylation, and moderate severity a greater proportion of the dimethylated material, while intermediate or less controlled conditions will likely result in mixtures of all species possible.

We expect that these new diacids will form polyesters, as do the non-methylated counterparts. These polyesters are different from known polyesters, in regard to the changed mass, crystallographic unit cell, hydrophobicity, electron donicity, size, and other aspects of the new diacids. For example, methylated polyesters based upon these new diacids may exhibit greater free volume, increased ductility, softness, and better "hand" and dyeability, as well as less water absorption, and different melting points and glass transition temperatures, all of which will be useful for particular polymer applications such as fiber, sheet, and engineering applications requiring particular sets of properties.

The disproportionation reaction is carried out under the pressure of gaseous carbon dioxide. The gaseous mixture may contain an inert gas or gases such as nitrogen, methane, or other gaseous paraffinic, olefinic, and aromatic hydrocarbons. In the case of a gas mixture, CO₂ is preferably present as at least about 10% of the mixture. The presence of oxygen should be avoided due to the fact that it can affect the yields. Suitable CO₂ pressures are from about 200 to 10,000 psig. Actual pressures depend upon the partial pressures of other gases present. A more preferred CO₂ pressure range is from about 350 to 1100 psig. To accelerate the reaction and suppress the occurrence of side reactions the reaction temperature is preferably about 450°C and the pressure is about 500 psig.

The following examples will serve to illustrate specific embodiments of the invention disclosed herein. These examples are intended only as a means of illustration and should not be construed as limiting the scope of the invention in any way. Those skilled in the art will recognize many variations that may be made without departing from the spirit of the disclosed invention.

### EXPERIMENTAL

Potassium acetate, which melts (Condensed Chemical Dictionary, 6th edition) at 292°C, was used as a molten reaction medium for the Henkel reaction of potassium benzoate with and without ZnO as catalyst. It was thought that since the salt melts at a lower temperature than the carbonate eutectics, it might be possible to perform the reaction at a lower temperature. However, little Henkel reaction was observed at temperatures significantly below 400°C. Unexpectedly, however, on examining the reaction products of the black fused mass formed from the potassium acetate salt and the naphthoic or benzoate potassium salts at ca. 400°C and above, a significant portion of methylated diacids was observed. Thus, from a reaction conducted at 450°C and 500 psi of CO₂ containing the following ingredients, 50g of potassium acetate, 5g of potassium phthalate, 1g of ZnO, a yield of about 50% of TPA and 20% of methylated TPA was unexpectedly observed. The methylated TPA was a mixture of 2-methyl 1,4 dicarboxy benzene, 2,5 dimethyl 1,4 dicarboxybenzene, 2,3,5 trimethyl 1,4 dicarboxy benzene, and 2,3,5,6 tetramethyl 1,4 dicarboxy benzene. The initial product appeared to be the 2-methyl species, however again surprisingly, the major components were the 2,5 dimethyl and the tetramethyl TPA (permethyl TPA). A possible explanation of this result is that the symmetrically di- or tetra- substituted salts are more stable or form particles which are more isolated from the reaction medium.

In subsequent work it was also shown that it was possible to form the four methylated acids above directly from terephthalic acid dipotassium salt by reaction in potassium acetate melt. It was also possible to form them from benzoate salts. It was further found to be possible to form methylated 2,6 - NDA from 2,6 - NDA or its Henkel reaction precursors.

## Claims

1. Process for the preparation of an aromatic diacid which comprises heating an alkali metal salt of an aromatic monocarboxylic acid or asymmetrical aromatic dicarboxylic acid under pressure with carbon dioxide gas in the presence of a catalyst at disproportionation reaction conditions including a temperature from about 350-500°C, said process comprising using as the reaction medium one or more molten salts, alone or in combination.

2. The process of Claim 1 wherein the aromatic diacid is purified terephthalic acid.

3. The process of Claim 1 wherein the aromatic diacid is 2,6 - naphthalene dicarboxylic acid.

4. The process of Claim 1 wherein the catalyst is selected from the group consisting of compounds of Group IB, IIB, VB, or VIIB of the Periodic Table.

5. The process of Claim 4 wherein the catalyst is selected from the group consisting of zinc compounds, cadmium compounds, and mercury compounds in the form of oxides, halides, sulfates, carbonates, and carboxylates of these metals.

6. The process of Claim 5 wherein the catalyst is a zinc compound.

7. The process of Claim 6 wherein the catalyst is ZnO.

8. The process of Claim 1 wherein the reaction conditions include a carbon dioxide pressure of from about 344 to 6,895 kPa (50 to 1000 psi).

9. The process of Claim 8 wherein the pressure is from about 2,758 to 4,137 kPa (400 to 600 psi).

10. The process of Claim 1 wherein the molten salts are selected from any molten salt known in the art.

11. The process of Claim 10 wherein the molten salts are selected from an alkali or alkaline earth metal salt, or a mixture of two or more of such salts, that have a sufficiently low melting point to dissolve the other components of the reaction mixture without thermal decomposition and which are sufficiently inert under the reaction conditions not to react with the other components of the reaction mixture.

12. The process of Claim 11 wherein the molten salt solvent may also have a minor amount of another salt, i.e., not an alkali or alkaline earth metal salt, preferably derived from the same acid anion.

13. The process of Claim 11 wherein the alkali or alkaline earth metal salts are derived from an inorganic or an organic acid.

14. The process of Claim 13 wherein the inorganic acids are selected from phosphoric acid and nitric acid.

15. The process of Claim 13 wherein the organic acids are selected from aliphatic carbo lic acids.

16. The process of Claim 14 wherein the salts are selected from sodium nitrate, potassium nitrate, mixtures of sodium and potassium nitrate, mixtures of sodium nitrate, potassium nitrate and ammonium nitrate, mixtures of sodium nitrate, potassium nitrate and calcium nitrate, and mixtures thereof.

17. The process of Claim 11 wherein the salt is a eutectic mixture of salts.

18. The process Claim 1 for preparing a composition of matter comprising a methylated diacid, useful as a polyester monomer, by reacting an alkali metal salt of an aromatic monocarboxylic acid or asymmetrical aromatic dicarboxylic acid at disproportionation/isomerization reaction conditions at a temperature in the range of 350-500°C, under pressure with carbon dioxide gas in the presence of a catalyst selected from compounds of Group IB, IIB, VB, or VIIB of the Periodic Table, wherein said reaction takes place in the presence of a reaction medium selected from one or more molten salts or mixtures thereof.

## Patentansprüche

1. Verfahren zur Herstellung einer aromatischen Disäure, welches das Erhitzen eines Alkalimetallsalzes einer aromatischen Monocarbonsäure oder einer asymmetrischen aromatischen Dicarbonsäure mit Kohlendioxidgas unter Druck in Gegenwart eines Katalysators bei Disproportionierungsreaktionsbedingungen, einschließlich einer Temperatur von etwa 350-500°C, umfasst, wobei das Verfahren die Verwendung - allein oder in Kombination - eines oder mehrerer Salzschmelzen als Reaktionsmedium umfasst.

2. Verfahren nach Anspruch 1, wobei die aromatische Disäure gereinigte Terephthalsäure ist.

3. Verfahren nach Anspruch 1, wobei die aromatische Disäure 2,6-Naphthalindicarbonsäure ist.

4. Verfahren nach Anspruch 1, wobei der Katalysator ausgewählt ist aus der Gruppe, bestehend aus Verbindungen der Gruppe IB, IIB, VB oder VIIB des Periodensystems.

5. Verfahren nach Anspruch 4, wobei der Katalysator ausgewählt ist aus der Gruppe, bestehend aus Zinkverbindungen, Cadmiumverbindungen und Quecksilberverbindungen in Form von Oxiden, Halogeniden, Sulfaten, Carbonaten und Carboxylaten dieser Metalle.

6. Verfahren nach Anspruch 5, wobei der Katalysator eine Zinkverbindung ist.

7. Verfahren nach Anspruch 6, wobei der Katalysator ZnO ist.

8. Verfahren nach Anspruch 1, wobei die Reaktionsbedingungen einen Kohlendioxiddruck von etwa 344 bis 6.895 kPa (50 bis 1000 psi) umfassen.

9. Verfahren nach Anspruch 8, wobei der Druck etwa 2.758 bis 4.137 kPa (400 bis 600 psi) beträgt.

10. Verfahren nach Anspruch 1, wobei die Salzschmelzen aus jeglichen, im Stand der Technik bekannten Salzschmelzen ausgewählt sind.

11. Verfahren nach Anspruch 10, wobei die Salzschmelzen ausgewählt sind aus einem Alkali- oder Erdalkalimetallsalz oder einer Mischung zweier oder mehrerer solcher Salze, die einen genügend niedrigen Schmelzpunkt aufweisen, um die anderen Bestandteile der Reaktionsmischung ohne thermische Zersetzung aufzulösen, und die unter den Reaktionsbedingungen genügend inert sind, um mit den anderen Bestandteilen der Reaktionsmischung nicht zu reagieren.

12. Verfahren nach Anspruch 11, wobei das Salzschmelzenlösungsmittel auch eine geringe Menge eines anderen Salzes, d.h. nicht eines Alkali- oder Erdalkalimetallsalzes, aufweisen kann, welches vorzugsweise von demselben Säureanion abgeleitet ist.

13. Verfahren nach Anspruch 11, wobei die Alkali- oder Erdalkalimetallsalze von einer anorganischen oder organischen Säure stammen.

14. Verfahren nach Anspruch 13, wobei die anorganischen Säuren aus Phosphorsäure und Salpetersäure ausgewählt sind.

15. Verfahren nach Anspruch 13, wobei die organischen Säuren aus aliphatischen Carbonsäuren ausgewählt sind.

16. Verfahren nach Anspruch 14, wobei die Salze ausgewählt sind aus Natriumnitrat, Kaliumnitrat, Mischungen von Natrium- und Kaliumnitrat, Mischungen von Natriumnitrat, Kaliumnitrat und Ammoniumnitrat, Mischungen von Natriumnitrat, Kaliumnitrat und Calciumnitrat und Mischungen davon.

17. Verfahren nach Anspruch 11, wobei das Salz eine eutektische Salzmischung ist.

18. Verfahren nach Anspruch 1 zur Herstellung einer Stoffzusammensetzung, umfassend eine als Polyestermonomer nützliche methylierte Disäure, durch Umsetzen eines Alkalimetallsalzes einer aromatischen Monocarbonsäure oder einer asymmetrischen aromatischen Dicarbonsäure mit Kohlendioxidgas unter Druck bei Disproportionierungs/Isomerisierungsreaktionsbedingungen und einer Temperatur im Bereich von 350-500°C in Gegenwart eines Katalysators, der ausgewählt ist aus Verbindungen der Gruppe IB, IIB, VB oder VIIB des Periodensystems, wobei die Reaktion in Gegenwart eines Reaktionsmediums stattfindet, welches ausgewählt ist aus einem oder mehreren Salzschmelzen oder Mischungen davon.

## Revendications

1. Procédé de préparation d'un diacide aromatique qui comprend le chauffage d'un sel de métal alcalin d'un acide monocarboxylique aromatique ou d'un acide dicarboxylique aromatique asymétrique sous pression avec du dioxyde de carbone gazeux en présence d'un catalyseur dans des conditions réactionnelles de dismutation comprenant une température de 350 - 500 °C, ledit procédé comprenant l'utilisation comme milieu réactionnel d'un ou plusieurs sels fondus, seul ou en combinaison.

2. Procédé selon la revendication 1, dans lequel le diacide aromatique est l'acide téréphtalique purifié.

3. Procédé selon la revendication 1, dans lequel le diacide aromatique est l'acide 2,6-naphtalènedicarboxylique.

4. Procédé selon la revendication 1, dans lequel le catalyseur est choisi dans le groupe constitué par des composés du groupe IB, IIB, VB, ou VIIB du tableau périodique.

5. Procédé selon la revendication 4, dans lequel le catalyseur est choisi dans le groupe constitué par des composés de zinc, des composés de cadmium, et des composés de mercure sous forme d'oxydes, d'halogénures, de sulfates, de carbonates, et de carboxylates de ces métaux.

6. Procédé selon la revendication 5, dans lequel le catalyseur est un composé de zinc.

7. Procédé selon la revendication 6, dans lequel le catalyseur est ZnO.

8. Procédé selon la revendication 1, dans lequel les conditions réactionnelles comprennent une pression du dioxyde de carbone d'environ 344 à 6,895 kPa (50 à 1000 psi).

9. Procédé selon la revendication 8, dans lequel la pression est d'environ 2,758 à 4,137 kPa (400 à 600 psi).

10. Procédé selon la revendication 1, dans lequel les sels fondus sont choisis parmi des sels quelconques connus dans la technique.

11. Procédé selon la revendication 10, dans lequel les sels fondus sont choisi parmi un sel de métaux alcalins ou alcalino-terreux, ou un mélange de deux ou plus de ces sels, qui ont un point de fusion suffisamment bas pour dissoudre les autres composants du mélange réactionnel sans décomposition thermique et qui sont suffisamment inertes dans les conditions réactionnelle pour ne pas réagir avec les autres composants du mélange réactionnel.

12. Procédé selon la revendication 11, dans lequel le sel solvant fondu peut également avoir une quantité mineure d'un autre sel, c'est-à-dire un sel qui n'est pas de métal alcalin ou alcalino-terreux, de préférence dérivé du même anion acide.

13. Procédé selon la revendication 11, dans lequel les sels de métaux alcalins ou alcalino-terreux sont dérivés d'un acide inorganique ou organique.

14. Procédé selon la revendication 13, dans lequel les acides inorganiques sont choisis parmi l'acide phosphorique et l'acide nitrique.

15. Procédé selon la revendication 13, dans lequel les acides organiques sont choisis parmi des acides carboxyliques aliphatiques.

16. Procédé selon la revendication 14, dans lequel les sels sont choisis parmi le nitrate de sodium, le nitrate de potassium, des mélanges de nitrate de sodium et de potassium, des mélanges de nitrate de sodium, de nitrate de potassium et de nitrate d'ammonium, des mélanges de nitrate de sodium, de nitrate de potassium et de nitrate de calcium, et des mélanges de ceux-ci.

17. Procédé selon la revendication 11, dans lequel le sel est un mélange eutectique de sels.

18. Procédé selon la revendication 1 de préparation d'une composition de matière comprenant un diacide méthylé, utile comme monomère polymère, en faisant réagir un sel de métal alcalin d'un acide monocarboxylique aromatique ou d'un acide dicarboxylique aromatique asymétrique dans des conditions réactionnelles de dismutation/isomérisation à une température dans la plage de 350 - 500 °C, sous pression avec du dioxyde de carbone gazeux en présence d'un catalyseur choisi parmi des composés du groupe IB, IIB, VB, ou VIIB du tableau périodique, dans lequel ladite réaction a lieu en présence d'un milieu réactionnel choisi parmi un ou plusieurs sels fondus ou les mélanges de ceux-ci.
